(19) Europäisches Patentamt
European Patent Office
Office européen des brevets

(11) Publication number: **0 338 092 A1**

(12) # EUROPEAN PATENT APPLICATION

published in accordance with Art. 158(3) EPC

(21) Application number: **88909366.2**

(22) Date of filing: **28.10.88**

Data of the international application taken as a basis:

(86) International application number:
**PCT/JP88/01100**

(87) International publication number:
**WO89/03684 (05.05.89 89/10)**

(51) Int. Cl.³: **A 61 K 31/70**
A 61 K 31/725

(30) Priority: **30.10.87 JP 276939/87**

(43) Date of publication of application:
**25.10.89 Bulletin 89/43**

(84) Designated Contracting States:
**DE FR GB IT**

(71) Applicant: **Chugai Seiyaku Kabushiki Kaisha**
**5-1, 5-chome, Ukima Kita-ku**
**Tokyo 115(JP)**

(72) Inventor: **TAKAGI, Michiro Chugai Seiyaku Kabushiki Kaisha**
**1-9, Kyobashi 2-chome**
**Chuo-ku Tokyo 104(JP)**

(72) Inventor: **WADA, Sakae Chugai Seiyaku Kabushiki Kaisha**
**41-8, Takada 3-chome**
**Toshima-ku Tokyo 171(JP)**

(72) Inventor: **KIMURA, Takao Chugai Seiyaku Kabushiki Kaisha**
**41-8, Takada 3-chome**
**Toshima-ku Tokyo 171(JP)**

(74) Representative: **Vossius & Partner**
**Siebertstrasse 4 P.O. Box 86 07 67**
**D-8000 München 86(DE)**

(54) **ANTI-HIV AGENT.**

(57) An anti-HIV agent which contains as an active ingredient an alkali metal salt or alkaline earth metal salt of sulfate of a compound composed of 4 to 10 D-glucose molecules linearly bound to each other through and α-1,4 bond and represented by general formula (I)

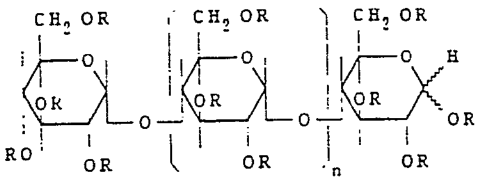

(I)

(wherein R represents a hydrogen atom or $-SO_3M$ (M:alkali metal or alkaline earth metal), and n represents an integer of 2 to 8, except the case where all R groups represent hydrogen atoms at the same time). The anti-HIV agent containing the above-described compound as an active ingredient can be used as a drug for treating acquired immunodeficiency syndrome (AIDS) or AIDS-related complex (ARC).

EP 0 338 092 A1

SPECIFICATION

TITLE OF THE INVENTION

ANTI-HIV AGENT

TECHNICAL FIELD

The present invention relates to an anti-HIV agent which contains as an effective ingredient an alkali metal or alkaline earth metal salt of an oligosaccharide poly-sulfate ester. More particularly, the present invention relates to an anti-HIV agent which contains as an effective ingredient an alkali metal or alkaline earth metal salt of a sulfate ester of an oligosaccharide in which 4 - 10 molecules of D-glucose are connected linearly by $\alpha$-1,4 bonds.

BACKGROUND ART

AIDS is a disease caused by infection with a human immunodeficiency virus (HIV) and in which the patient suffers from a deficiency in the body's immunity to disease and will eventually die from opportunistic infections caused by viruses or bacteria or from malignant tumors. Azidothymidine (AZT) is currently used as an effective drug against AIDS but it has been found to cause adverse side effects such as strong toxicity to the bone marrow. There has therefore been a strong demand for the development of other drugs.

Sulfate esters of saccharides such as dextran have been known to be useful as anti-arteriosclerotic agents. It has also been reported recently that sulfate esters of various saccharides display the ability to inhibit the replication of HIV *in vitro* using cultured human lymphocytic cells (European Patent Publication Nos. 232,744 and 240,098). According to European Patent Publication No. 232,744, the anti-HIV activities of saccharide sulfate esters are not merely associated with their molecular weights but it also states that dextran sulfate is useful when its molecular weight is at least 5,000.

According to European Patent Publication No. 240,098, all the saccharide sulfate esters that have satisfactory anti-HIV activity have molecular weights of at least 5,000. However, as the molecular weights of saccharide sulfate esters increase, their absorption becomes very low when

orally administered, and in order to attain the desired therapeutic effects, they have to be administered in high doses. Unfortunately, however, this inevitably causes side effects. The present inventors, therefore, conducted further studies on the applicability of saccharide sulfate esters as anti-HIV agents and found that improved anti-HIV effects could be exhibited by alkali metal or alkaline earth metal salts of sulfate esters of lower molecular weight saccharides, in particular, those in which 4 - 10 molecules of D-glucose are connected linearly by α-1,4 bonds. The anti-HIV agent of the present invention has a molecular weight of no higher than 5,000 and, compared to sulfate esters of higher-molecular weight saccharides such as a sodium salt of sulfate ester of dextrin having a molecular weight of 7,000 - 8,000, this anti-HIV agent, when sodium is taken as an exemplary alkali metal, has outstanding features in that it not only has high absorbability when orally administered but also exhibits enhanced anti-HIV activity, and yet that it causes reduced cytotoxicity. The saccharides that are specifically supported by the examples of European Patent Publication No. 232,744 and 240,098 are dextran, pentosan, chondroitin, heparin, etc. and the saccharides within the scope of the present invention are not described in any part of these references.

DISCLOSURE OF THE INVENTION

The present invention relates to an anti-HIV agent which contains as an effective ingredient an alkali metal or alkaline earth metal salt of a sulfate ester of an oligosaccharide, in particular, one in which 4 - 10 molecules of D-glucose are connected linearly by α-1,4 bonds. The anti-HIV agent of the present invention is a compound represented by the following general formula (1):

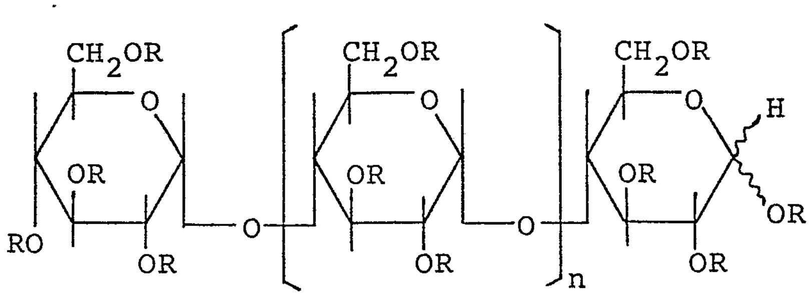

(I)

where R is a hydrogen atom or $-SO_3M$ (M is an alkali metal or an alkaline earth metal) and n is an integer of 2 to 8, provided that not all of the Rs present are hydrogen atoms.

Preferred examples of the compounds of the present invention which are represented by the general formula (I) include: sulfate esters of pentasaccharide, hexasaccharide and heptasaccharide, such as maltopentaose, maltohexaose and maltoheptaose, as well as sulfate esters of the acid hydrolyzate of dextrin, their average molecular weight being in the range of from about 1,000 to about 4,500, preferably from about 2,000 to about 3,500.

The anti-HIV activities of the sulfate esters of oligosaccharides of the present invention also depend, to a by no means small extent, on the number of sulfonic acid substituents. With saccharides of comparatively high molecular weights, their sulfate esters exhibit satisfactory anti-HlV activities even if they contain a fairly small number of sulfonic acid substituents. However, with saccharides of comparatively low molecular weights, their sulfate esters are unable to exhibit high anti-HIV activities unless they contain a large number of sulfonic acid substituents. To take sodium as an exemplary alkali metal, the preferred number of sulfonic acid substituents is at least 16%, most preferably at least 18%, as expressed in terms of the sulfur content (the proportion of the overall molecular weight taken by sulfur atoms).

Depending upon the specific type of sulfonation method employed, the sulfate esters of oligosaccharides prepared in accordance with the present invention will differ in the position and number of sulfonic acid substituents. Mixtures of these sulfonate esters are also useful as anti-HIV agents within the scope of the present invention.

If dextrin is used as the starting material to be sulfonated, sulfate esters of saccharides having different degrees of polymerization will form and these can also be used in combination as anti-HIV agents in the scope of the present invention. The dextrins used in this instance preferably have D.E. (dextrose equivalent) values in the

range of from 11 ± 1 to 42 ± 1.

The alkali metal or alkaline earth metal salts of sulfate esters of oligosaccharides according to the present invention contain sodium, potassium, magnesium, etc. as alkali metals or alkaline earth metals, with sodium and potassium being particularly preferred.

BRIEF DESCRIPTION OF THE DRAWINGS

Figs. 1 - 6 are graphs showing the results of an experiment conducted in order to investigate the ability of sodium salts of oligosaccharide poly-sulfate esters of the present invention to inhibit the cytopathy of HIV infected cells.

BEST MODE FOR CARRYING OUT THE INVENTION

The sulfate esters of oligosaccharides of the present invention are stable and can be formulated in various dosage forms by standard methods. Preferred dosage forms include tablets, granules, capsules and injections.

The sulfate esters of oligosaccharides of the present invention may be administered in doses that vary with the severity of disease and the route of administration. For oral administration, an appropriate dose may be selected from the range of 100 - 1,000 mg per adult per day.

Hereinunder, the present invention will be described more specifically by way of preparation examples and experiment. However, the present invention is in no way restricted by those examples and the experiment.

Preparation Example 1

A mixture of pyridine (60 ml) and a pyridine-$SO_3$ complex (12 g) was heated to 50°C and after addition of 2 g of dextrin (D.E value: 42 ± 1), the mixture was stirred at 65°C for 4 - 5 hours. A syrupy substance precipitated and it acquired color as the reaction proceeded. After completion of the reaction, the reaction mixture was cooled and freed of pyridine by decantation. The residual syrupy substance was dissolved in ca. 50 ml of water. One or two drops of a phenolphthalein solution were added and carbon dioxide gas was blown into the mixture under stirring with an excess of calcium hydroxide being added. The

precipitating inorganic salt was removed by filtration and the filtrate, after being concentrated to ca. 20 ml, was passed through an ion-exchange resin IR-120 (Na form). The effluent was concentrated to a syrupy state and thereafter reduced to a powder form in cold ethanol. After filtration, the powder was dried to obtain a sodium salt of a dextrin sulfate ester in an amount of 5.01 g. This product had an average molecular weight of ca. 960.

Elemental analysis for $(C_{12}H_{14}O_{10})\cdot(SO_3Na)_6\cdot 3H_2O$:

Cal'd: C 14.17, H 1.99, S 18.89

Found: C 14.00, H 2.11, S 18.85

Preparation Examples 2 - 4

Starting with the acid hydrolyzates of dextrins having different D.E. values, the procedures described in Preparation Example 1 were repeated so as to prepare sodium salts of dextrin sulfate esters having the physical data shown in Table 1 below.

Table 1

| Run No. | D.E. value of starting dextrin | Product | | | | |
|---|---|---|---|---|---|---|
| | | Elemental analysis | | | | Average mol. wt. |
| | | | C | H | S | |
| 2 | 24 ± 1 | cal'd* | 14.17 | 1.99 | 18.89 | 1900 |
| | | found | 13.90 | 2.16 | 19.19 | |
| 3 | 18 ± 1 | cal'd* | 14.17 | 1.99 | 18.89 | 2400 |
| | | found | 14.04 | 2.16 | 19.26 | |
| 4 | 11 ± 1 | cal'd** | 14.41 | 1.91 | 19.06 | 4300 |
| | | found | 14.90 | 2.33 | 18.76 | |

* : for $(C_{12}H_{14}O_{10})\cdot(SO_3Na)_6\cdot 3H_2O$

** : for $(C_{12}H_{14}O_{10})\cdot(SO_3Na)_6\cdot 2\frac{1}{2}H_2O$

Preparation Example 5

A mixture of pyridine (80 ml), a pyridine-$SO_3$ complex (14 g) and maltohexaose (2 g) was treated by the procedures described in Preparation Example 1 so as to prepare a sodium

salt of maltohexaose sulfate ester (mol. wt. 2,678) in an amount of 4.2 g.

m.p. 201 - 204°C (with decomposition)

Elemental analysis for $(C_{36}H_{46}O_{31})\cdot(SO_3Na)_{16}\cdot 3H_2O$:

Cal'd: C 16.15, H 1.96, S 19.16

Found: C 16.10, H 2.29, S 19.30

Preparation Examples 6 and 7

Starting with maltopentaose and maltoheptaose, the procedures described in Preparation Example 1 were repeated to prepare a sodium salt of maltopentaose sulfate ester (Preparation Example 6) and a sodium salt of maltoheptaose sulfate ester (Preparation Example 7).

6. Na salt of maltopentaose sulfate ester (mol. wt. 2,293)

Elemental analysis for $(C_{30}H_{38}O_{26})\cdot(SO_3Na)_{14}\cdot 2H_2O$:

Cal'd: C 15.71, H 1.85, S 19.57

Found: C 15.90, H 2.22, S 19.50

7. Na salt of maltoheptaose sulfate ester (mol. wt. 3,164)

Elemental analysis for $(C_{42}H_{53}O_{36})\cdot(SO_3Na)_{19}\cdot 4H_2O$:

Cal'd: C 15.94, H 1.94, S 19.26

Found: C 15.83, H 2.21, S 19.38

Experiment

(1) Materials

The sodium salts of oligosaccharide poly-sulfate esters prepared in Preparation Examples 1 - 3 and 5 - 7 were used as test samples. Molt-4 clone No. 8 cells were infected with a virus obtained from Molt-4/HIV producing cell.

(2) Method

The experiment was conducted in accordance with the method of Itoh et al. described in Antiviral Research, 7 (1987), 361 - 367.

a) Inhibition of cytopathy of HIV infected cells

HIV viruses were adsorbed on Molt-4 clone 8 cells and the test samples were added at various concentrations. Four days later, the liquid culture diluted test samples were replaced, and the number of viable and dead cells were counted after 6 days. As a control, non-infected Molt-4 clone No. 8 cells were subjected to the same

experiment. The results are shown in Figs. 1 - 6, in which ■ and □ denote the infected and non-infected cells, respectively, and the Sample Numbers are keyed to the Preparation Example Numbers.

b) Detection of viral antigen

The proportions of HIV antigen positive cells were determined by an indirect fluorescent antibody technique using an anti-HIV antibody positive serum as antibody against the infected cells. The results are shown in Tables 2 and 3, in which each of the figure listed denotes the percentage of infected cells taken by HIV antigen positive cells.

Table 2

| Sample No. \ Amount of sample added (μg/ml) | 0 | 3.9 | 15.6 | 62.5 | 250 | 1000 |
|---|---|---|---|---|---|---|
| 1 | 87.0 | 26.7 | 6.4 | 6.8 | 6.0 | 6.5 |
| 2 | 86.8 | 24.6 | 20.0 | 18.0 | 9.2 | 4.9 |
| 3 | 89.2 | 15.5 | 7.0 | 4.8 | 7.5 | 6.9 |

Table 3

| Sample No. \ Amount of sample added (μg/ml) | 0 | 7.8 | 32 | 125 | 500 | 1000 |
|---|---|---|---|---|---|---|
| 5 | 92.6 | 20.3 | 5.3 | 5.8 | 3.6 | 4.4 |
| 6 | 96.3 | 24.1 | 6.4 | 6.0 | 4.4 | 4.6 |
| 7 | 89.8 | 18.2 | 4.8 | 4.4 | 4.1 | 3.2 |

(3) Results

a) Inhibition of cytopathy of HIV infected cells

As Figs. 1 - 6 show, sample Nos. 1 - 3 and Nos. 5 - 7 showed very strong cytopathy inhibiting effects at doses of 3.9 μg/ml and 7.8 μg/ml, respectively. None of the

samples tested caused detectable cytotoxicity even when they were applied in doses of up to 1,000 μg/ml.

b) Inhibition of viral antigen production

As Table 2 shows, sample Nos. 1 - 3 showed low levels of antigen positivity in the range of 15.5 - 26.7% at a dose of 3.9 μg/ml, and much lower levels at doses of 15.6 μg/ml and above. This data, which demonstrates the strong inhibition of viral antigen production, was substantially reproduced with sample Nos. 5 - 7, as is apparent from Table 3.

INDUSTRIAL APPLICABILITY

The anti-HIV agent of the present invention is useful as a therapeutic agent for the treatment of acquired immunodeficiency syndrome (AIDS) or AIDS-related complex (ARC). Given the rapid spread of these syndromes on a worldwide basis, the anti-HIV agent of the present invention is expected to be effectively utilized in the area of pharmaceutical industry.

CLAIMS

1. An anti-HIV agent containing as an effective ingredient an alkali metal or alkaline earth metal salt of a sulfate ester of an oligosaccharide in which 4 - 10 molecules of D-glucose are connected linearly by α-1,4 bonds.

2. An anti-HIV agent according to Claim 1 which is an alkali metal or alkaline earth metal salt of a sulfate ester of maltopentaose, maltohexaose or maltoheptaose.

3. An anti-HIV agent according to Claim 1 wherein the alkali metal or alkaline earth metal salt of a sulfate ester of oligosaccharide is an alkali metal or alkaline earth metal salt of a sulfate ester of the acid hydrolyzate of dextrin.

4. An anti-HIV agent according to Claim 3 wherein the acid hydrolyzate of dextrin has a D. E. (dextrose equivalent) value in the range of from 11 ± 1 to 42 ± 1.

5. An anti-HIV agent according to any one of Claims 1 to 4 wherein the alkali metal or alkaline earth metal is selected from the group consisting of sodium, potassium calcium and magnesium.

6. An anti-HIV agent according to any one of Claims 1 to 5 wherein the alkali metal is sodium.

7. An anti-HIV agent according to Claim 6 which has a sulfur content of at least 16%, preferably at least 18%.

Fig. 1

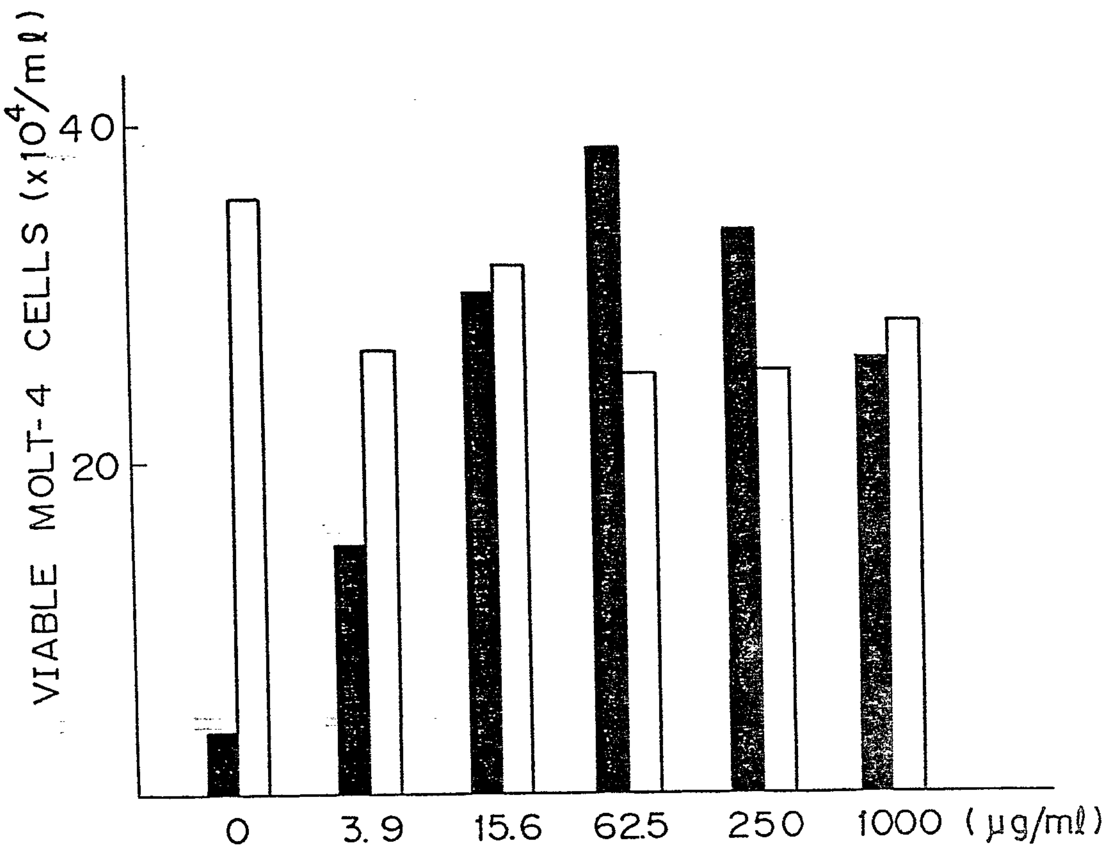


TEST SAMPLE- 1

# Fig. 2

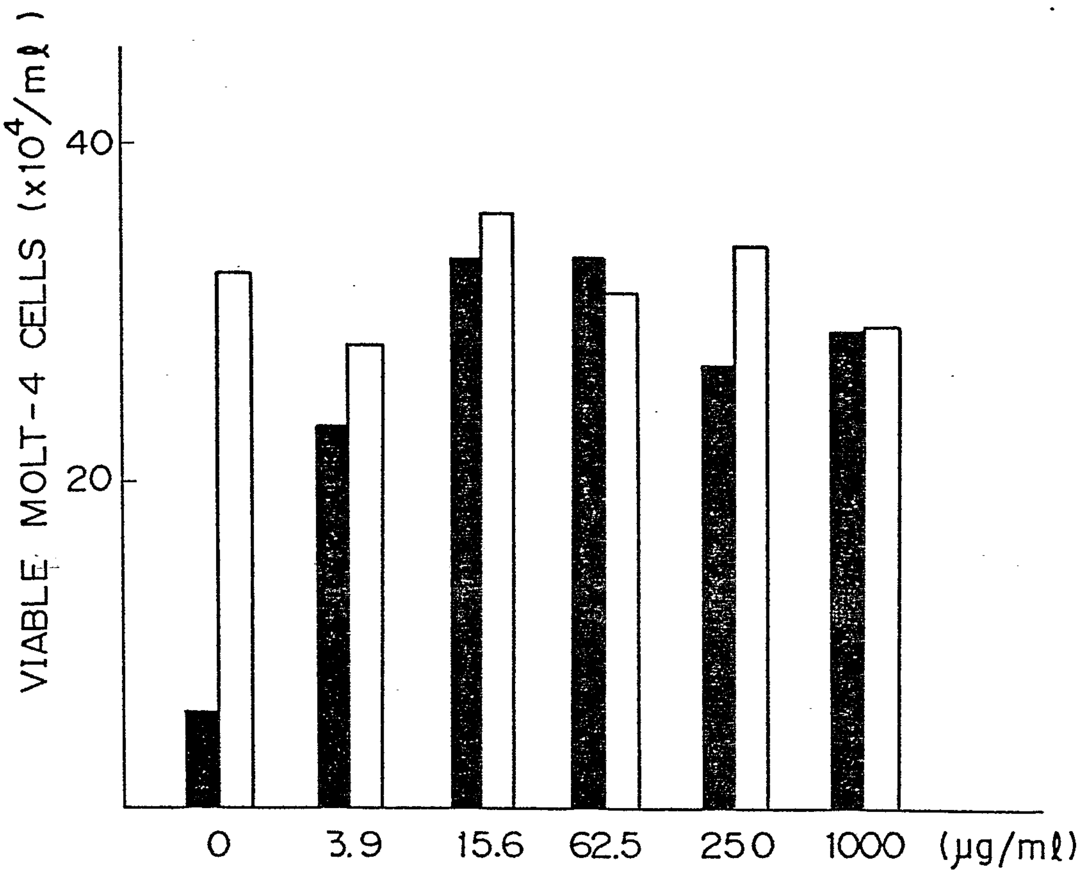


TEST SAMPLE- 2

Fig. 3

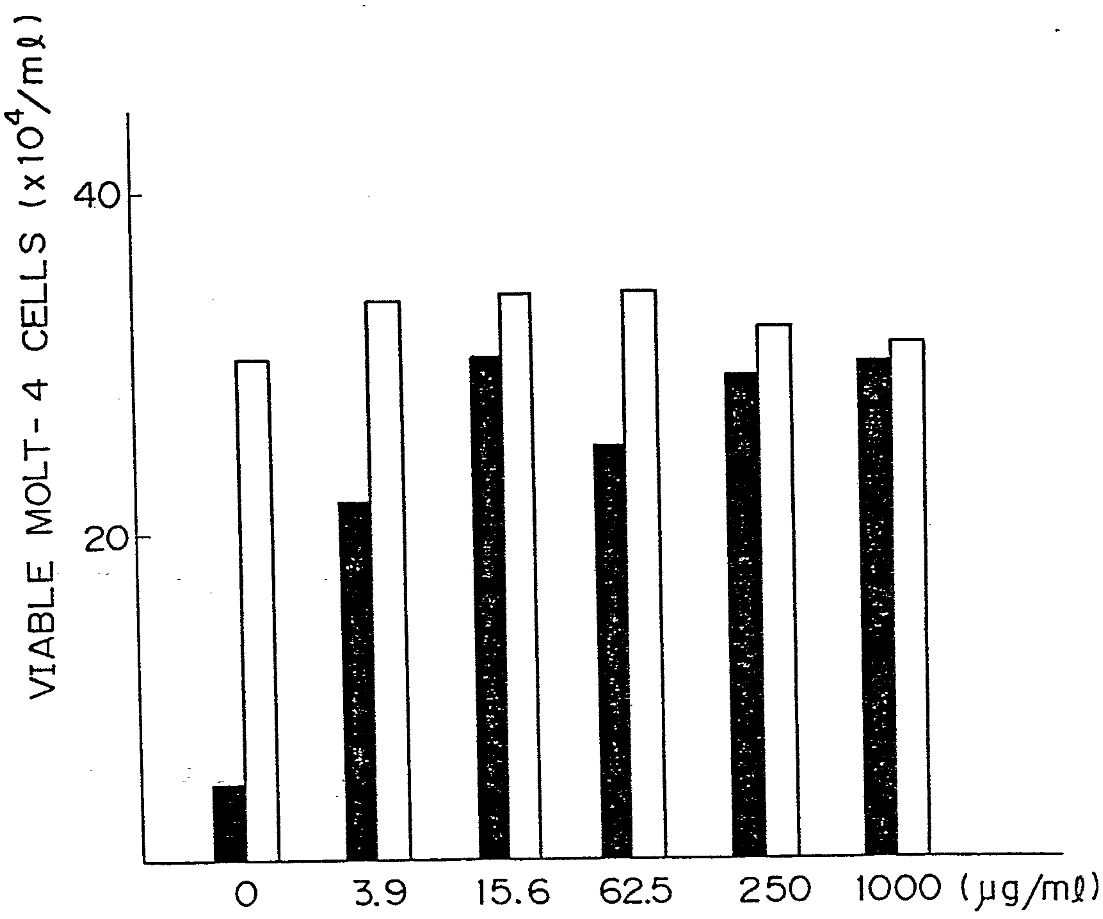


TEST SAMPLE-3

Fig. 4

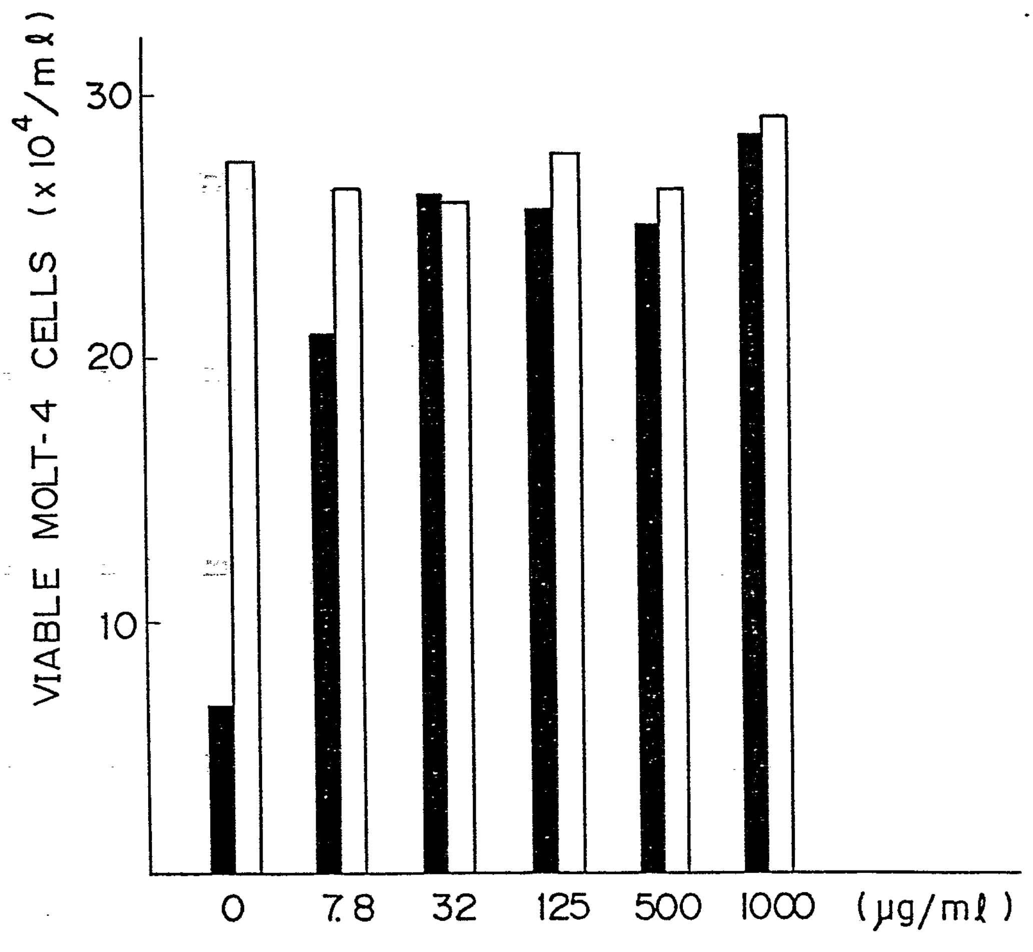


TEST SAMPLE-5

# Fig. 5

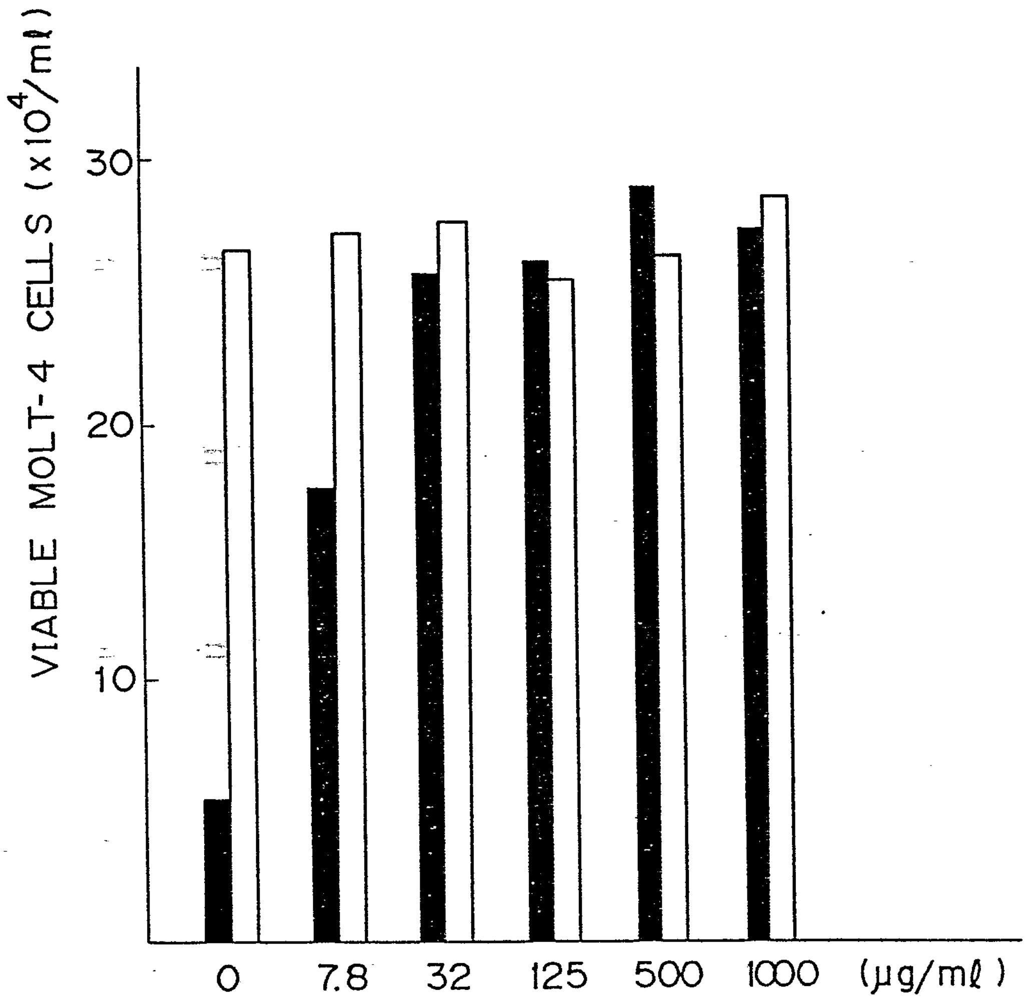


TEST SAMPLE-6

Fig. 6

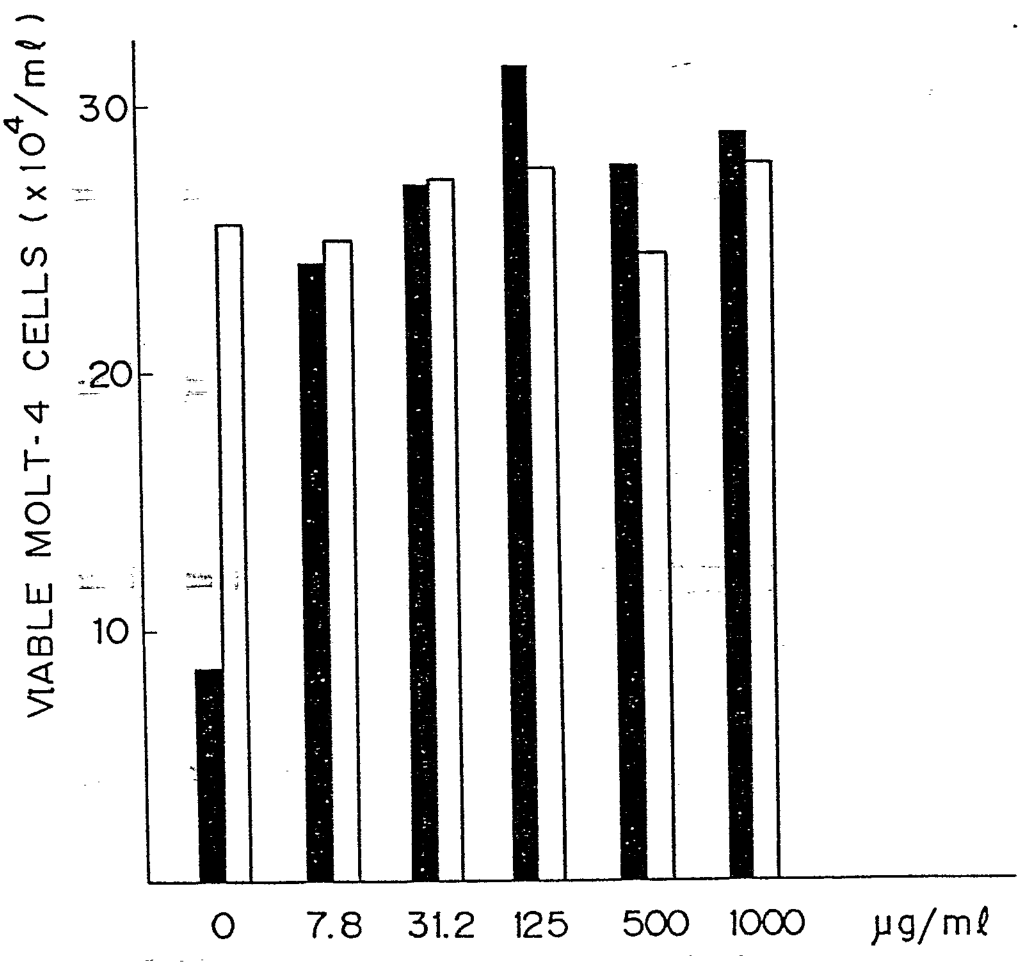


TEST SAMPLE-7

# INTERNATIONAL SEARCH REPORT

International Application No PCT/JP88/01100

**I. CLASSIFICATION OF SUBJECT MATTER** (if several classification symbols apply, indicate all) [6]

According to International Patent Classification (IPC) or to both National Classification and IPC

Int.Cl[4] A61K31/70, A61K31/725

**II. FIELDS SEARCHED**

Minimum Documentation Searched [7]

| Classification System | Classification Symbols |
|---|---|
| IPC | A61K31/70, A61K31/725, C07H11/00, C08B37/00 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | DE, A, 3601136 (Max-Planck-Gesellshaft zur Foerderung der Wissenschaften e.V.) 23. Jul. 1987 (23. 07. 87) & EP, A, 232744 & JP, A, 62-215529 | 1-7 |
| A | EP, A, 240098 (Kabushiki Kaisha Ueno Seiyaku Oyo Kenkyusho) 7. Oct. 1987 (07. 10. 87) & AU, A, 8771074 & JP, A, 63-045223 & ZA, A, 8702359 | 1-7 |
| A | Hoppe-Seyler's Z. Physiol. Chem., vol. 357(4), p. 499-508(1976), Schaffrath, Dieter; Stuhlsatz, Helmut W.; Greiling, Helmut: "Interactions of glycosaminoglycans with DNA and RNA synthesizing enzymes in vitro" (Chemical Abstracts, Abstract No. 85(3): 16134n) | 1-7 |
| A | Cancer Res., vol. 38(8), p.2401-7(1978), DiCioccio, Richard A.; Srivastava, B.I. Sahai: "Inhibition of deoxynucleo tide- | 1-7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 16, 1988 (16. 11. 88) | December 5, 1988 (05. 12. 88) |
| **International Searching Authority** | **Signature of Authorized Officer** |
| Japanese Patent Office | |

International Application No.

FURTHER INFORMATION CONTINUED FROM THE SECOND SHEET

| | | |
|---|---|---|
| | polymerizing enzyme activities of human cells and of simian sarcoma virus by heparin" (Chemical Abstracts, Abstract No. 89(25): 208939h) | |
| A | J. Nat. Cancer Inst., vol. 51(5), p.1705-7 (1973) Kiehl, Bryan L.; Stott, Phillip B.; Huang, Andrew U.; Buthala, Darwin A.: "Anticoagulants as inhibitors of reverse transcriptase activity" (Chemical Abstracts, Abstract No. 81(1): 286v) | 1-7 |

V.☐ OBSERVATIONS WHERE CERTAIN CLAIMS WERE FOUND UNSEARCHABLE[10]

This international search report has not been established in respect of certain claims under Article 17(2) (a) for the following reasons:

1.☐ Claim numbers............, because they relate to subject matter[11] not required to be searched by this Authority, namely:

2.☐ Claim numbers............, because they relate to parts of the international application that do not comply with the prescribed requirements to such an extent that no meaningful international search can be carried out[12], specifically:

VI.☐ OBSERVATIONS WHERE UNITY OF INVENTION IS LACKING[11]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

**FURTHER INFORMATION CONTINUED FROM THE SECOND SHEET**

| | | |
|---|---|---|
| A | EP, A, 66379 (RIKER Lab. Inc.) 8. Dec. 1982 (08. 12. 82) & JP, A, 58-918 & PT, A, 74899 & ZA, A, 8203366 | 1-7 |

**V. ☐ OBSERVATIONS WHERE CERTAIN CLAIMS WERE FOUND UNSEARCHABLE[10]**

This International search report has not been established in respect of certain claims under Article 17(2) (a) for the following reasons:

1. ☐ Claim numbers..........., because they relate to subject matter[12] not required to be searched by this Authority, namely:

2. ☐ Claim numbers..........., because they relate to parts of the International application that do not comply with the prescribed requirements to such an extent that no meaningful International search can be carried out[13], specifically:

**VI. ☐ OBSERVATIONS WHERE UNITY OF INVENTION IS LACKING[11]**

This International Searching Authority found multiple inventions in this International application as follows:

1. ☐ As all required additional search fees were timely paid by the applicant, this International search report covers all searchable claims of the International application.
2. ☐ As only some of the required additional search fees were timely paid by the applicant, this International search report covers only those claims of the International application for which fees were paid, specifically claims:
3. ☐ No required additional search fees were timely paid by the applicant. Consequently, this International search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.